# EUROPÄISCHE PATENTSCHRIFT

(11) **EP 0 730 007 B1**
(45) Veröffentlichungstag und Bekanntmachung des Hinweises auf die Patenterteilung: **23.08.2000**
(21) Anmeldenummer: 96102514.5
(22) Anmeldetag: 20.02.1996
(51) Int. Cl.: C09B 23/10, C09B 23/14, C09B 57/02, C07B 43/08, C07C 253/00, C09B 57/00

(54) **Cyanierung von Doppelbindungssystemen**
Cyanation of systems containing double bonds
Cyanuration de doubles liaisons

(30) Priorität: 03.03.1995 DE 19507415; 14.03.1995 DE 19509043
(43) Veröffentlichungstag der Anmeldung: 04.09.1996
(73) Patentinhaber: BAYER AG, 51368 Leverkusen (DE)
(72) Erfinder: Hamprecht, Rainer, Dr., 51519 Odenthal (DE); Müller, Claudia, Dr., 51061 Köln (DE)

(56) Entgegenhaltungen:
- EP-A- 0 027 529
- EP-A- 0 065 743
- GB-A- 2 006 253

## Beschreibung

Die vorliegende Erfindung betrifft ein Verfahren zur Cyanierung von Doppelbindungssystemen.

In Ep-A-27529 werden Cyangruppen-haltige Cumarine und ein Verfahren zu ihrer Herstellung offenbart, das ausgehend von den Cyangruppen-freien Cumarinen durch Umsetzung mit einem Cyanid und anschliessender Oxidation gekennzeichnet ist.
Die Oxidation erfolgt dabei vorzugsweise mit Bleitetraacetat (sh. Beispiele 1-4) und für spezielle Cumarine auch mit anderen Oxidationsmitteln (sh. Beispiel. 2).

Aus DE-A 2 844 299 (GB-A-2 006 253) ist bereits ein Verfahren zur Cyanierung von Doppelbindungssystemen beschrieben, wobei das als Oxidationsmittel insbesondere verwendete Brom gewisse anwendungstechnische Nachteile aufweist. Diese zu überwinden war die Aufgabe der vorliegenden Erfindung.

Es wurde nun ein Verfahren zur Herstellung von Verbindungen der Formel I gefunden, 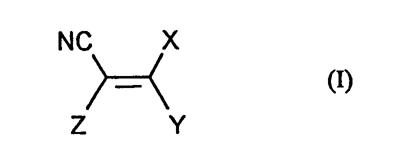 worin
- X und Y: unabhängig voneinander für einen elektronenanziehenden Rest stehen,
oder
- X und Y: gemeinsam mit dem Kohlenstoffatom, an welches sie gebunden sind, einen gegebenenfalls substituierten, heterocyclischen oder carbocyclischen Ring bilden, wobei der carbocyclische Rest vorzugsweise 5-7 gliedrig ist und gegebenenfalls substituiert und/oder eine oder mehrere Carbonylgruppen enthält,
- Z: für gegebenenfalls substituiertes, gegebenenfalls anelliertes Aryl oder für einen gegebenenfalls substituierten gegebenenfalls anellierten Heterocyclus steht,
oder
- Y und Z: gemeinsam mit den Kohlenstoffatomen, an welche sie gebunden sind und unter Einschluß der Doppelbindung einen gegebenenfalls substituierten Heterocyclus bilden,
dadurch gekennzeichnet ist, daß man Verbindungen der Formel 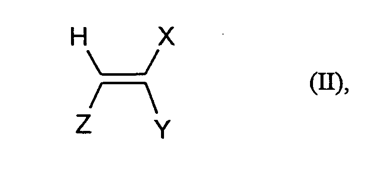 worin
- X, Y und Z: die oben angegebene Bedeutung haben,
mit einem Cyanid oder einer Cyanid-abgebenden Verbindung und anschließend mit einem von Br₂ verschiedenen Oxidationsmittel in Gegenwart von Halogeniden umsetzt.

Vorzugsweise stehen X und Y unabhängig voneinander für einen Rest, dessen Hammett'sche Substituentenkonstante σ (para) > 0 ist.

Eine entsprechende Auflistung von Hammett'schen Substituentenkonstanten findet sich z.B. in Sykes, Reaktionsmechanismen der organischen Chemie, 9. Aufl. Weinheim, VCH Verlagsgesellschaft, 1988, oder kann nach bekannten Verfahren bestimmt werden.

Eine bevorzugte Ausführungsform des erfindungsgemäßen Verfahrens ist dadurch gekennzeichnet, daß man Verbindungen der Formel II mit einem Cyanid oder einer Cyanid-abgebenden Verbindung und anschließend mit einem von Br₂ verschiedenen Oxidationsmittel in Gegenwart von Halogeniden und Säure umsetzt.

Vorzugsweise dient das erfindungsgemäße Verfahren zur Herstellung von Verbindungen der Formel I, worin
- X: -CN, -CO-OV₁, 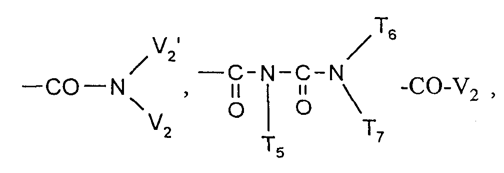 -SO₂V₁, 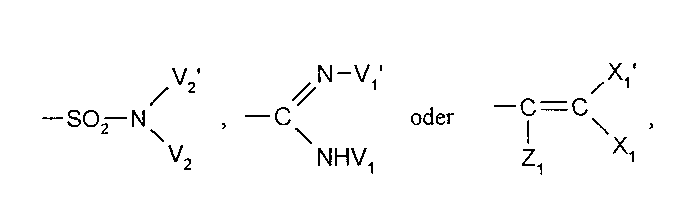 bedeutet, wobei
V₁ und V₁' unabhängig voneinander für Alkyl, Cycloalkyl, Alkenyl, Aralkyl oder Aryl stehen,
T₅, T₆ und T₇ unabhängig voneinander Wasserstoff, unsubstituiertes oder substituiertes Aryl, insbesondere Phenyl bedeuten,
V₂ und V₂' unabhängig voneinander für H oder V₁ stehen, oder V₁ und V₁' bzw. V₂ und V₂' jeweils gemeinsam mit den Resten, an welche sie gebunden sind, für einen 5- oder 6-gliedrigen, gegebenenfalls substituierten ungesättigten, vorzugsweise aromatischen Heterocyclus stehen, der 1 bis 3 gleiche oder verschiedene Heteroatome aus der Reihe O, N, S enthält und der gegebenenfalls durch einen substituierten oder unsubstituierten Benzolring anelliert ist, oder für einen 5- oder 6-gliedrigen, gegebenenfalls substituierten ungesättigten Carbocyclus, vorzugsweise Phenyl, stehen,
X₁ und X₁' unabhängig voneinander für , 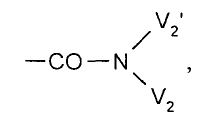 -CO-V₂, -SO₂V₁, 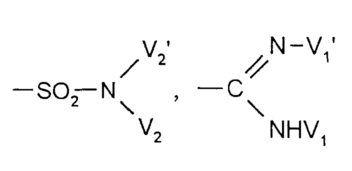 stehen, wobei
V₁, V₁', V₂ und V₂' die oben angegebene Bedeutung haben, oder
X₁ und X₁' gemeinsam mit dem C-Atom, an das sie gebunden sind für einen 5- oder 6-gliedrigen, gegebenenfalls substituierten Heterocyclus, der 1 bis 2 N-Atome enthält und der gegebenenfalls durch einen weiteren 5-gliedrigen ungesättigten Heterocyclus oder einen Benzolring anelliert ist, stehen,
- Z₁: für gegebenenfalls substituiertes C₆-C₁₀-Aryl, das gegebenenfalls durch einen gesättigten oder ungesättigten Heterocyclus anelliert ist, oder für einen ungesättigten 5- oder 6-gliedrigen, gegebenenfalls substituierten, Heterocyclus, der 1 bis 4 gleiche oder verschiedene Heteroatome aus der Reihe O, N, S enthält und der durch einen Benzolring anelliert sein kann, steht,
oder
- Z₁ und X₁: gemeinsam mit den Kohlenstoffatomen, an welche sie gebunden sind, und unter Einschluß der Doppelbindung einen gegebenenfalls substituierten und gegebenenfalls anellierten Heterocyclus oder einen aromatischen, carbocyclischen Ring, insbesondere- Benzolring, bilden,
- Y: gleiches oder verschiedenes X bedeutet oder X und Y gemeinsam mit dem C-Atom, an das sie gebunden sind, für einen 5- oder 6-gliedrigen gegebenenfalls substituierten Heterocyclus, der 1 bis 2 N-Atome enthält, und der gegebenenfalls durch einen weiteren 5-gliedrigen ungesättigten Heterocyclus oder einen Benzolring anelliert sein kann, stehen,
- Z: für gegebenenfalls substituiertes C₆-C₁₀-Aryl, das gegebenenfalls durch einen gesättigten oder ungesättigten Heterocyclus anelliert sein kann, oder für einen ungesättigten 5- oder 6-gliedrigen gegebenenfalls substituierten Heterocyclus, der 1 bis 4 gleiche oder verschiedene Heteroatome aus der Reihe O, N, S enthält und der durch einen Benzolring anelliert sein kann, steht,
oder
- Y und Z: gemeinsam, unter Einschluß der Doppelbindung an welche sie gebunden sind, einen 6-gliedrigen, gegebenenfalls substituierten Heterocyclus bilden, welcher ein Heteroatom aus der Reihe O, N, S enthält und welcher gegebenenfalls durch einen substituierten oder unsubstituierten Benzolring anelliert ist,
wobei als Substituenten für einen Carbocyclus und einen Heterocyclus insbesondere Alkyl, Alkylsulfonyl, Aryl, Aralkyl, Alkenyl, -SO₂OR₁, Cycloalkyl, Alkoxy, Alkoxycarbonyl, Acyl, Halogen, Cyan, C=O, C=NH, jeweils gegebenenfalls substituiertes Carbonamid oder Sulfonamid, Nitro oder exocyclisches =O, =NH, in Frage kommen und
für den anellierten Benzolring als Substituenten insbesondere Alkyl, SO₂-Alkyl, gegebenenfalls -SO₂NR₁R₂, substituiertes Sulfonamid, Aryl, Aralkyl, Cycloalkyl, Alkoxy, Alkoxycarbonyl, Acyl, Halogen, Cyan, Nitro oder Alkenyl in Frage kommen.
Als Substituenten für den aromatischen Carbocyclus oder den Heterocyclus in der Definition von Z kommen insbesondere 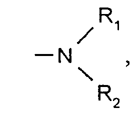 OR₁, NHCOR₁ und NHSO₂R₁ in Frage, wobei
- R₁ und R₂: unabhängig voneinander für Wasserstoff, gegebenenfalls substituiertes Alkyl, Alkenyl, Cycloalkyl, Aralkyl oder Aryl stehen, oder R₁ und R₂ zusammen mit dem Stickstoffatom, an das sie gebunden sind, einen 5- bis 6-gliedrigen Heterocyclus mit 1 bis 3 Heteroatomen der Reihe O, N, S bilden.

Das erfindungsgemäße Verfahren dient vorzugsweise zur Herstellung von Verbindungen der Formel 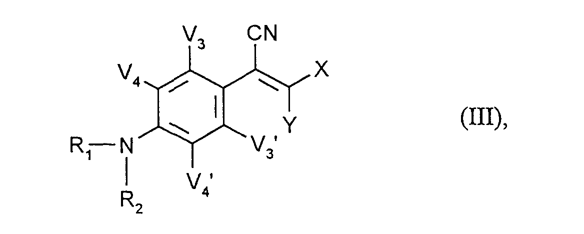 worin
- X, Y, R₁ und R₂: die vorgenannte Bedeutung haben,
- V₃ und V₃': unabhängig voneinander für H, C₁-C₄-Alkyl, C₁-C₄-Alkoxy, -Cl, Br, -OCO-C₁-C₄-Alkyl stehen,
- V₄ und V₄': unabhängig voneinander für H, C₁-C₄-Alkyl oder C₁-C₄-Alkoxy stehen,
oder
V₃ und V₄ unter Einschluß der Ring-C-Atome, an welche sie gebunden sind, einen 6-gliedrigen Carbocyclus bilden,
und/oder
V₃' und V₄' unter Einschluß der Ring-C-Atome, an welche sie gebunden sind, einen 6-gliedrigen Carbocyclus bilden,
und/oder
R₁ und/oder R₂ mit V₄ und/oder V₄' zusammen die restlichen Glieder eines Tetrahydro- oder Dihydropyridin- bzw. Chinolizinringes bilden.

Besonders bevorzugt werden nach dem erfindungsgemäßen Verfahren Verbindungen der Formel (I) hergestellt, bei denen Y und Z unter Einschluß der Doppelbindung einen Cumarinring bilden. Diese Verbindungen entsprechen der Formel (IV) 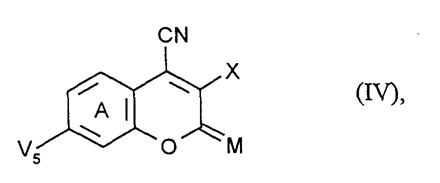 in welcher
- V₅: für 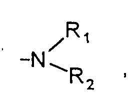 OR₁, NHCOR₁ oder NHSO₂R₁ steht,
- R₁ und R₂: unabhängig voneinander für Wasserstoff, gegebenenfalls substituiertes Alkyl, Cycloalkyl, Aralkyl oder Aryl stehen, oder R₁ und R₂ zusammen mit dem Stickstoffatom, an das sie gebunden sind, und gegebenenfalls unter Einschluß weiterer Heteroatome einen 5- bis 7-gliedrigen Heterocyclus bilden, oder
einer der Reste R₁ oder R₂ mit einem zur Aminogruppe o-ständigen Kohlenstoffatom des Ringes A einen ankondensierten, gesättigten, gegebenenfalls substituierten 5- oder 6-Ring bildet,
- M: für =NR₃, =NCOR₄ 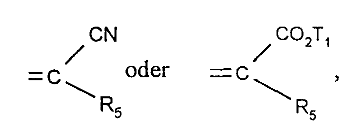 insbesondere
für =NH oder =O steht, wobei
R₃ für Alkyl oder gegebenenfalls substituiertes Phenyl,
R₄ für gegebenenfalls substituiertes Alkyl, Aralkyl, Aryl, Vinyl, Alkoxy, Phenoxy oder Amino und
R₅ für Carbalkoxy, Cyano oder gegebenenfalls substituiertes Carbonamid steht, oder
R₅ zusammen mit dem Substituenten X die restlichen Glieder eines ungesättigten 6-gliedrigen, gegebenenfalls anellierten N-Heterocyclus bildet,
und
- X: die oben angegebene Bedeutung hat.

Als elektronenanziehende Reste X kommen vor allem in Frage:
-CN, -COOV₁, worin V₁ für Alkyl, insbesondere für C₁-C₆-Alkyl steht, 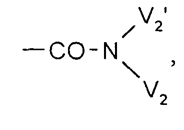 worin V₂ und V₂' die oben angegebene Bedeutung haben,
-SO₂V₁, worin V₁ für Alkyl oder Aryl steht, oder einer der folgenden Reste:
a) 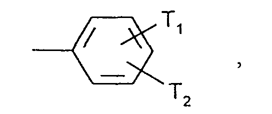 wobei
   - T₁: für Cyan oder Nitro und
   - T₂: für Wasserstoff, Cyan, Halogen, Alkylsulfonyl, gegebenenfalls substituiertes, Sulfonamid, insbesondere durch Alkyl substituiert oder Nitro steht,
b) 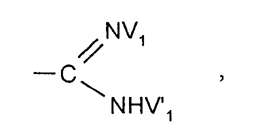 wobei
   - V₁ und V₁': unabhängig voneinander einen gegebenenfalls substituierten C₁-C₁₀-Alkyl-, C₃-C₇-Cycloalkyl-, C₆-C₁₀-Ar-C₁-C₁₀-alkyl- oder C₆-C₁₀-Arylrest bedeuten,
c) 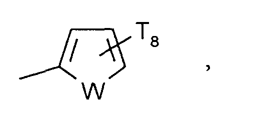 wobei
   - T₈: für Wasserstoff, Alkoxycarbonyl, gegebenenfalls substituiertes Carbonamid oder Cyano steht und
   - W: für Sauerstoff oder Schwefel steht,
d) 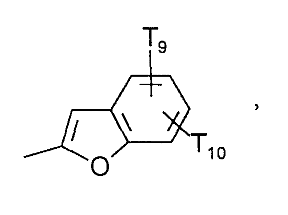
e) 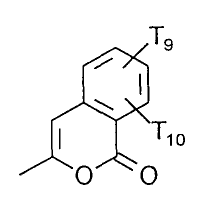 und
f) 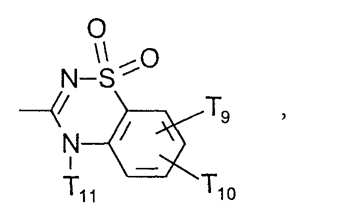 wobei in den Formeln d), e) und f)
   - T₉: für Wasserstoff, Alkyl, Chlor, Brom oder Alkoxy,
   - T₁₀: für Wasserstoff oder Alkyl und
   - T₁₁: für Acyl- oder einen gegebenenfalls substituierten C₁-C₄-Alkyl- oder Phenylrest, vorzugsweise aber für Wasserstoff, steht,
g) einen gegebenenfalls substituierten insbesondere anellierten Pyrazol-, Imidazol-, Thiazol-, Oxazol-, 1,2,4-Triazol-, 1,3,4-Oxadiazol-, 1,3,4-Thiadiazol-, Chinoxalon-, Chinazolon-, Benzimidazol-, Benzoxazol-, Benzthiazol-, Pyridin-, Chinolin- oder Pyrimidinring, der in Nachbarstellung zu einem Ring-Stickstoffatom mit dem Cumarinring verbunden ist, wie z.B. 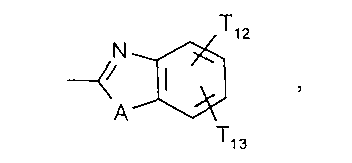 wobei
   - A: für Sauerstoff, Schwefel oder die Gruppierung 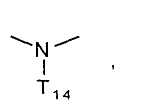
   - T₁₄: für Wasserstoff, Alkyl oder Aralkyl,
   - T₁₂: für Wasserstoff, Alkyl, Halogen, Alkoxy, NHCOR₁, NHSO₂R₁,
   - T₁₃: für Wasserstoff oder Alkyl steht oder
   T₁₂ und T₁₃ zusammen auch einen weiteren ankondensierten gegebenenfalls substituierten aromatischen Ring bilden können; bevorzugt sind Benzthiazol-, Benzoxazol- oder Benzimidazolreste, worin der Benzolrest gegebenenfalls durch Cl, -NHSO₂CH₃ oder CH₃ weitersubstituiert ist und wobei T₁₄ zusammen mit R₅ die restlichen Glieder eines N-Heterocyclus bilden kann,
h) 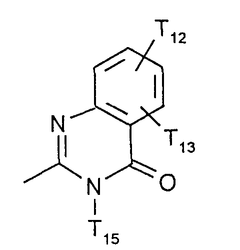 und
i) 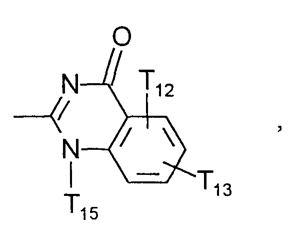 wobei
   - T₁₂ und T₁₃: die oben angegebene Bedeutung haben und
   - T₁₅: Wasserstoff oder gegebenenfalls substituiertes Alkyl oder Phenyl ist,
   und
j) 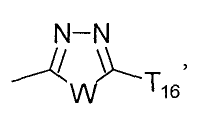 wobei
   - W: Sauerstoff oder Schwefel ist und
   - T₁₆: gegebenenfalls substituiertes Alkyl, Cycloalkyl, Aralkyl, Phenyl, Pyridyl bedeutet.

Die Substituenten V₁, V₁', T₁-T₁₆, V oder Z in der Bedeutung einer Alkylgruppe können gleich oder verschieden, geradkettig oder verzweigt sein, insbesondere handelt es sich um niedrigmolekulare Alkylgruppen mit 1 bis 7 und vorzugsweise 1 bis 4 Kohlenstoffatomen, wie Methyl, Ethyl, Propyl, Isopropyl, tert.-Butyl, Amyl, Hexyl, Heptyl oder um Alkylgruppen mit längerer Kette, wie Octyl, Decyl oder Dodecyl. Als Substituenten kommen z.B. die folgenden in Betracht: Hydroxyl, niedrigmolekulares Alkoxy oder Carbalkoxy, Phenoxy, Cyano, Carbonamido, Halogen, insbesondere Chlor oder Brom, Acetoxy. Vorteilhaft bedeuten R₁ und R₂ Methyl oder Ethyl. V₁, V₁', T₁-T₁₆, V oder Z stellen in der Bedeutung einer Cycloalkylgruppe insbesondere die Cyclohexyl- oder Methylcyclohexylgruppe und in der Bedeutung einer Aralkylgruppe vor allem die Benzyl-, Phenethyl- oder β-Phenyl-β-hydroxy-ethylgruppe dar.

Bilden R₁ und R₂ oder Z₁ und Z₂ zusammen mit dem Stickstoffatom und gegebenenfalls unter Einschluß weiterer Heteroatome, wie O, S oder N, einen 5- bis 7-gliedrigen Heteroring, so handelt es sich z.B. um den Piperidin-, Pyrrolidin-, Morpholin-, Triazol-, Piperazin- oder N-Methyl-piperazinring.

Bilden R₁ oder R₂ zusammen mit dem Stickstoff einen in ortho-Stellung zum Stickstoff des Ringes A ankondensierten Rest, so handelt es sich vorzugsweise um folgende Gruppierungen:

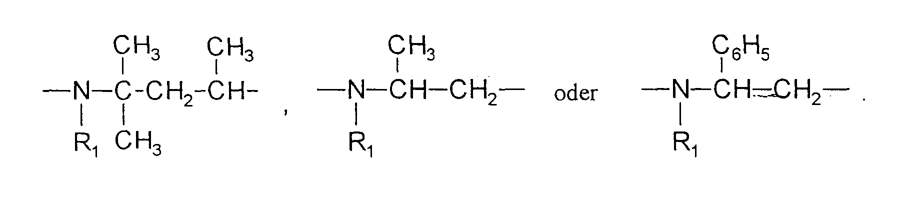

Bedeuten R, T₁-T₁₆ oder Z einen Arylrest, so handelt es sich z.B. um den Naphthalin- und vorzugsweise um den Phenylrest.

Als Substituenten an den Aryl-, insbesondere Phenyl- wie auch Benzoresten kommen in Betracht: ein oder mehrere Alkylgruppen, wie Methyl, Ethyl oder Isopropyl, Alkoxygruppen, wie Methoxy oder Ethoxy, Acylaminogruppen, wie Acetylamino oder Benzoylamino, Halogenatome, wie Chlor oder Brom, Hydroxy, Cyan, Rhodan, Amino, mono- und di-Alkylamino, Phenylamino, N-Phenyl-N-alkylamino, Phenyl, Phenoxy, Nitro, Acyl oder Acyloxy, wie Acetyl oder Acetoxy. Bevorzugt sind Methyl oder Ethyl.

Die substituierten Carbonamid- oder Sulfonamidgruppen enthalten vorzugsweise 1 bis 10 Kohlenstoffatome und sind vorzugsweise N-Methyl-, N,N-Dimethyl-, N-Ethyl-, N,N-Diethyl-, N-Benzyl- und N-Phenylcarbonamid oder -sulfonamid.

Zur Durchführung des erfindungsgemäßen Verfahrens sind prinzipiell eine Vielzahl von organischen und anorganischen Cyaniden und Cyanid-abgebenden Verbindungen geeignet. Insbesondere geeignet sind Cyanide der 1. und 2. Haupt- und Nebengruppe des Periodensystems der Elemente sowie Ammonium- und durch Alkyl, Aryl, Aralkyl substituierte Ammoniumcyanide. Als Beispiele seien genannt:
Ca(CN)₂, Zn(CN)₂, NH₄CN, Na[Cu(CN)₂], (n-C₄H₉)₄NCN und insbesondere KCN und NaCN.

Als Cyanide kommen auch Cyanid-abspaltende Verbindungen wie z.B. Hydroxyacetonitril, Nitromethan, Formaldehydoxim in Frage. Es ist weiterhin möglich, die genannten Cyanide in beliebigen Gemischen untereinander einzusetzen.

Üblicherweise werden zur Durchführung des erfindungsgemäßen Verfahrens äquivalente Mengen an Cyaniden eingesetzt. Zuweilen ist es jedoch vorteilhaft, einen Überschuß von 1 bis 100 % einzusetzen. Die Cyanide können in fester Form oder auch gelöst in einem Lösungsmittel wie z.B. Wasser, Methanol, Ethanol, DMF, NMP, Dimethylacetamid, Caprolactam eingesetzt werden. Bevorzugt ist der Einsatz der Cyanide in wäßriger Lösung.

Als Lösungsmittel zur Durchführung des erfindungsgemäßen Verfahrens eignen sich prinzipiell polare und unpolare Lösungsmittel. Als polare Lösungsmittel seien beispielsweise genannt: Protische Lösungsmittel, wie z.B. Wasser und Alkohole und aprotische Lösungsmittel, wie z.B. Dimethylformamid, N-Methylpyrrolidon, Aceton und Dioxan, wobei folgende polare Lösungsmittel bevorzugt sind: Wasser, Alkohole, Pyridin, Picolin, Dimethylformamid, N-Methylpyrrolidon, Dimethylsulfoxid, Acetonitril. Die polaren Lösungsmittel können in beliebigen Gemischen untereinander eingesetzt werden.

Als unpolare Lösungsmittel seien genannt: Alkane, Toluol, Xylol, Chlorbenzol, Dichlorbenzol und Dichlormethan.

Bevorzugt ist der Einsatz polarer Lösungsmittel, insbesondere im Gemisch mit Wasser. Besonders hervorzuheben sind folgende Mischungen: Dimethylformamid/ Wasser, N-Methylpyrrolidon/Wasser sowie Dimethylsulfoxid/Wasser.

In einigen Fällen kann es sich als vorteilhaft erweisen, dem polaren oder dem unpolaren Lösungsmittel, bzw. dem Gemisch aus Wasser und einen weiteren polarem Lösungsmittel, einen Phasentransferkatalysator zuzusetzen.

Als Phasentransferkatalysatoren kommen z.B. in Frage: Quaternäre Ammoniumsalze, Phosphoniumsalze, Polyethylenglykol, Kronenether, Amine und Phosphoniumverbindungen.

Der Phasentransferkatalysator wird üblicherweise in Mengen von 0,1 bis 2 Mol-Äquivalenten, bezogen auf eingesetztes Edukt, vorzugsweise von 0,5 bis 1,5 Mol-Äquivalenten eingesetzt.

Die Reaktionstemperaturen für diesen ersten Teilschritt liegen bei 0 bis 100°C, vorzugsweise bei 20 bis 80°C. Das bei diesem Teilschritt entstehende Zwischenprodukt kann unter geeigneten Schutzmaßnahmen (z.B. Wasserausschluß) isoliert werden, wird aber vorzugsweise direkt umgesetzt.

Geeignete Säuren sind organischer oder anorganischer Art, vorzugsweise Carbonsäuren und besonders bevorzugt Mineralsäuren. Als Beispiele seien genannt: Essigsäure, Salzsäure, Schwefelsäure, Phosphorsäure, Bromwasserstoffsäure, Iodwasserstoffsäure.

Die Menge der eingesetzten Säure ist breit variierbar, vorzugsweise zwischen 10 und 200 Mol-%, bezogen auf das Ausgangsmaterial der Formel (II), vorzugsweise wird jedoch eine äquivalente Menge eingesetzt.

Geeignete Halogenide sind organische und anorganische Halogenverbindungen wie z.B. Chloride, Bromide und Iodide von Alkalimetallen, von diesen ist Bromid bevorzugt. Neben organischen Halogeniden wie z.B. Tetraalkylammoniumhalogeniden kommen aus Gründen von Zugänglichkeit, Kosten, Entsorgung und Löslichkeit bevorzugt anorganische Halogenide in Frage. Von diesen sind die Halogenide der 1. und 2. Hauptgruppe des Periodensystems bevorzugt, unter diesen wiederum die der 1. Hauptgruppe. Beispiele sind NaCl, NaBr, KCl und KBr, aber auch FeCl₃, ZnCl₂, ZnBr₂, MgCl₂ oder NH₄Br. Die Menge des einzusetzenden Halogenids beträgt 0,1 bis 200 Mol-%, bezogen auf Edukt (II), vorzugsweise 10 bis 100 %.

Das Halogenid kann vorteilhafterweise auch in einer katalytischen Menge von nur 0,1 bis 0,6 Mol-%, bezogen auf Edukt (II), eingesetzt werden. Dabei sind geringere Einsatzkosten an Halogenid und geringere Belastung der Mutterlauge nach der Reaktion mit Halogenid hervorzuheben.

Die bei dem erfindungsgemäßen Verfahren in Frage kommenden Oxidationsmittel sind von Br₂ verschieden. Abgesehen von dieser Maßgabe kommen prinzipiell alle Oxidationsmittel, insbesondere solche mit einem chemischen Potential von mindestens 0,1 eV, vorzugsweise größer 0,4 eV in Frage. Beispielhaft seien genannt: Persäuren, Persulfate, Peroxide, Chloranil, Salpetersäure, Peroxid-Anlagerungsverbindungen wie z.B. Perborat oder Luft-Sauerstoff in Gegenwart geeigneter Katalysatoren wie beispielsweise V₂O₅. Das Oxidationsmittel kann in Substanz oder als Lösung, vorzugsweise als wäßrige Lösung, eingesetzt werden.

Bevorzugte Oxidationsmittel sind Wasserstoffperoxid und die davon abgeleiteten Verbindungen. Wasserstoffperoxid wird bevorzugt in wäßriger, verdünnter oder konzentrierter Form eingesetzt werden. Bevorzugt werden 20 bis 50 % wäßrige Lösungen. Die Menge des Oxidationsmittel ist vorzugsweise äquivalent zum Edukt der Formel (II), es können auch Überschüsse bis zu 50 % nützlich sein. Die genannten Oxidationsmittel können auch im Gemisch untereinander eingesetzt werden.

Die Reaktionstemperatur für den Teilschritt der Oxidation liegt im allgemeinen bei -20°C bis 150°C, vorzugsweise 0 bis 100°C, besonders bevorzugt bei 20 bis 90°C.

Die Verbindungen (II) sind bekannt (vgl. DE-A-2 844 299) oder können auf bekannte Art und Weise hergestellt werden.

Zwei besonders bevorzugte Verfahrensvarianten werden nachfolgenden beschrieben:

Nach Variante A versetzt man eine Lösung bzw. Suspension des Cyanidanlagerungsproduktes der Formel (IV) 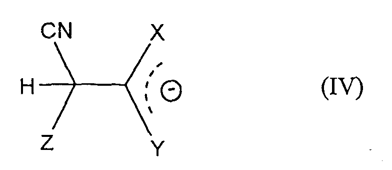 mit der Säure, fügt das Halogenid und dann das Oxidationsmittel zu.

Nach Variante B werden Säure, Halogenid und Oxidationsmittel vorzugsweise in einem Lösungsmittel vorgelegt und das Cyanidanlagerungsprodukt (IV) zugefügt. Dieses Verfahren bietet den Vorteil, daß die Konkurrenzreaktion der Rückspaltung von (IV) zu (II) unterdrückt wird.

In einer Verfeinerung von Variante B werden Säure, Halogenid und nur ein Teil (z.B. 5 bis 50 %) des Oxidationsmittels vorgelegt und das Cyanidanlagerungsprodukt (IV) und der Rest des Oxidationsmittels gleichzeitig zugegeben.

In einer weiteren Verfeinerung von Variante B wird die Zugabe des Oxidationsmittels über eine Redoxelektrode so gesteuert, daß durch Kontrolle des Redox potentials immer ein Überschuß des Oxidationsmittels vorhanden ist.

Das erfindungsgemäße Verfahren ermöglicht z.B. die großtechnische Synthese von Farbstoffen, die sich hervorragend zum Färben oder Bedrucken von synthetischem und halbsynthetischem Textilmaterial z.B. aus Polyamiden, Triacetat und insbesondere Polyestern, sowie zum Färben von Kunststoffen in der Masse eignen. Nach dem erfindungsgemäßen Verfahren sind ebenfalls leicht sublimierbare Farbstoffe für den Transferdruck auf textile und nichttextile Substrate wie z.B. Cellulose-triacetat, Polyacrylnitril und insbesondere Polyestermaterialien herstellbar. Daneben können die nach dem erfindungsgemäßen Verfahren hergestellten Farbstoffe auch im D2T2-Prozeß (Dye-Diffusion-Thermo-Transfer) eingesetzt werden.

Erfindungsgemäß ist daher ebenfalls ein Verfahren zum Färben oder Bedrucken von synthetischen oder halbsynthetischen Textilmaterial, das Färben von Kunststoffen in der Masse zum Transferdruck auf textile und nichttextile Substrate sowie zum Thermotransfer, dadurch gekennzeichet, daß man Farbstoffe verwendet, die nach dem erfindungsgemäßen Herstellungsverfahren erhalten werden. Die Erfindung betrifft weiterhin Substrate die nach einem der obigen Druck- oder Färbeverfahren mit den erfindungsgemäß hergestellten Farbstoffe erhalten wurden.

Nach dem erfindungsgemäßen Verfahren erhält man die Farbstoffe in ausgezeichneter Reinheit und mit sehr guten Ausbeuten.

Die folgenden Beispiele sollen das erfindungsgemäße Verfahren erläutern, ohne es jedoch darauf zu beschränken.

### Beispiel 1

70,2 g des Farbstoffs der Formel 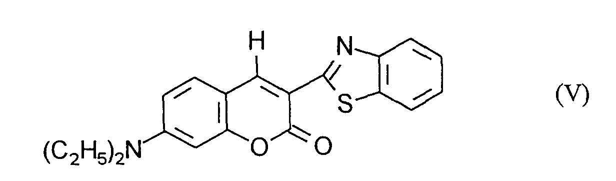 wurden in 352 ml N-Methylpyrrolidon (NMP) suspendiert. Hierzu wurden 10,8 g Natriumcyanid, gelöst in 18 ml Wasser, zugefügt. Die Temperatur stieg auf etwa 35°C. Man rührte ca. 1 h bei 45°C nach. Dabei ging der Farbstoff in Lösung. 10,4 g Natriumbromid wurden in 200 ml N-Methylpyrrolidon mit 20,4 g H₂SO₄ (96 %ig) und 21,4 g Wasserstoffperoxid (34 %ig) vorgelegt. Dazu tropfte man die Lösung des Cyanid-Anlagerungsproduktes. Die Temperatur stieg dabei auf ca. 70°C an. Nach dem Abkühlen wurde der Farbstoff filtriert und mit Methanol und Wasser gewaschen. Nach dem Trocknen erhielt man 70,9 g des Farbstoffes der Formel

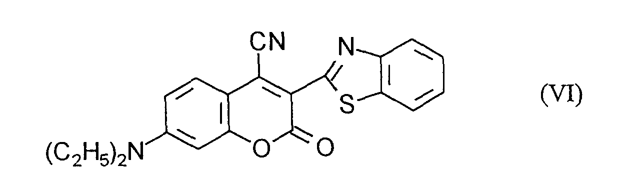

### Beispiel 2

350,5 g des Farbstoffs der Formel (V) gemäß Beispiel 1 wurden in 1760 ml N-Methylpyrrolidon suspendiert. Bei 20 bis 25°C wird eine Lösung von 53,9 g NaCN in 90 ml Wasser zugegeben. Man rührte 1 h bei 40 bis 45°C nach. Dabei ging der Farbstoff in Lösung. Die Lösung wird bei 0°C mit 51,5 g Natriumbromid, 56 ml H₂SO₄ (96 %ig) und 94,2 ml Wasserstoffperoxid (35 %ig) versetzt. Dabei stieg die Temperatur auf 80 bis 85°C an. Der Farbstoff (VI) wurde dann analog Beispiel 1 isoliert. Man erhält 361,4 g.

### Beispiel 3

Verwendete man anstatt N-Methylpyrrolidon DMF und verfuhr sonst gleich wie in Beispiel 1 beschrieben, erhielt man 70,2 g Farbstoff (VI).

### Beispiel 4

Verwendete man anstatt N-Methylpyrrolidon DMF und verfuhr sonst gleich wie in Beispiel 2 beschrieben, erhielt man 360 g Farbstoff (VI).

### Beispiel 5

Zu einer Suspension von 185 g des Farbstoffs der Formel 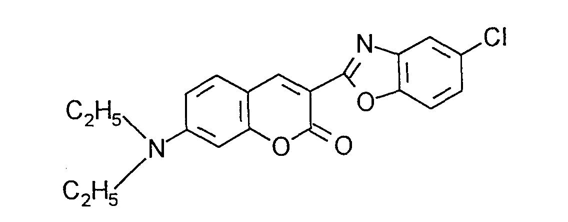 in 1 l N-Methylpyrrolidon wurde eine Lösung von 27 g Natriumcyanid in 46 ml Wasser getropft. Man ließ 2 Stunden bei 35°C nachrühren.

Zu einer Suspension von 57 g Natriumbromid in 500 ml N-Methylpyrrolidon (NMP) tropfte man bei 20°C 30,5 ml 96 %ige Schwefelsäure und anschließend 5 ml 35 %iges Wasserstoffperoxid. Im Verlauf einer Stunde ließ man gleichzeitig die Lösung des Cyanidanlagerungsproduktes und 42 ml 35 %iges Wasserstoffperoxid unter Rühren und Kühlen bei 25°C zulaufen. Man ließ 30 Minuten nachrühren, entfernte den Wasserstoffperoxidüberschuß mit wenigen ml Natriumbisulfitlösung, stellte mit 60 ml 30 %iger Natronlauge neutral und saugte den Farbstoff ab. Man wusch mit Methanol und Wasser und trocknete bei 50°C. Ausbeute 180 g; Fp. 234 bis 234,5°C.

### Beispiel 6

Verwendete man Natriumbromid in katalytischer Menge von 2,8 g und verfuhr sonst gleich wie in Beispiel 1 oder 2 beschrieben, verlängerte sich die Reaktionszeit um ca. 30 Minuten bis 2 h. Die Ausbeute war vergleichbar.

### Beispiel 7

Verwendete man statt katalytischer Mengen Natriumbromid äquimolare Mengen oder einen Überschuß an Natriumbromid (ca. 1-200 %) und verfuhr sonst gleich wie in Beispiel 1 oder 2 beschrieben erhielt man vergleichbare Ausbeuten.

Zu gleichen Ergebnissen gelangte man, wenn man statt Natriumbromid wie in Beispiel 1 bis 7 beschrieben Kaliumbromid, Lithiumbromid, Ammoniumbromid, Natriumiodid, Kaliumiodid, Lithiumiodid oder Natriumchlorid in ähnlichen Mengenverhältnissen wie in Bsp. 1-7 beschrieben verwendete.

Analog zu Beispiel 1 lassen sich die folgenden Verbindungen herstellen:

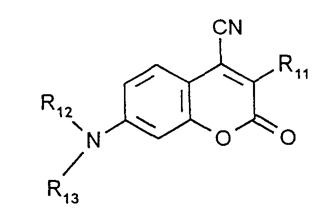

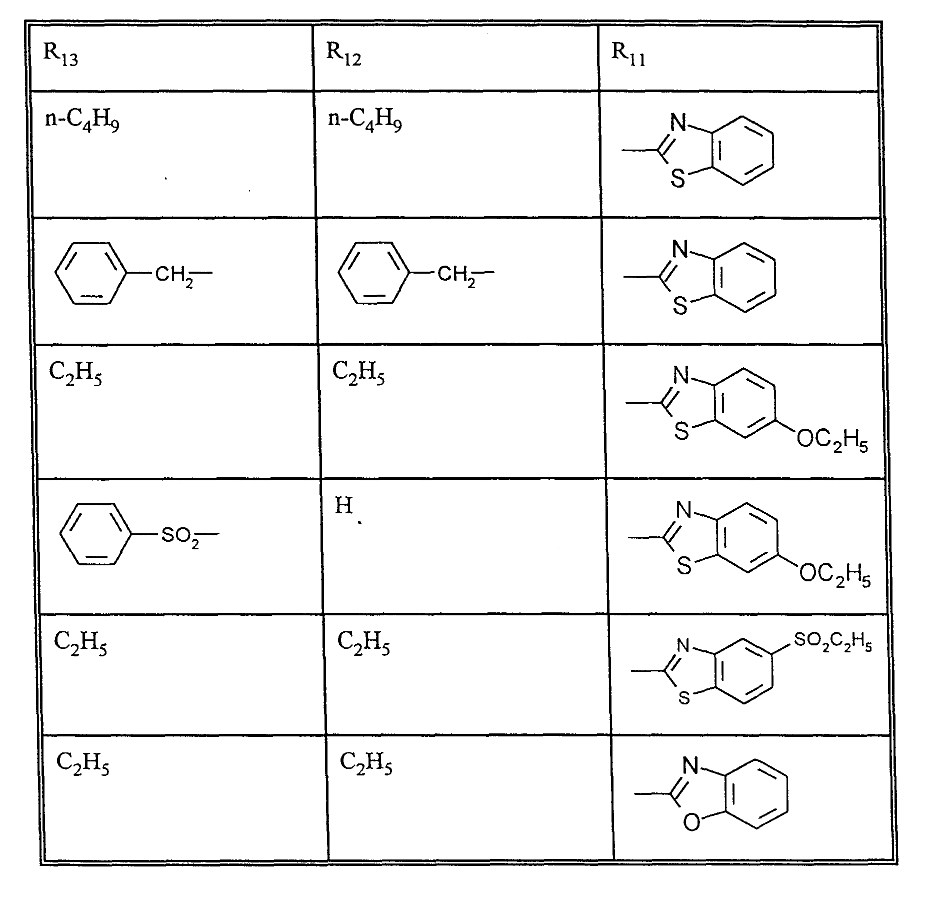

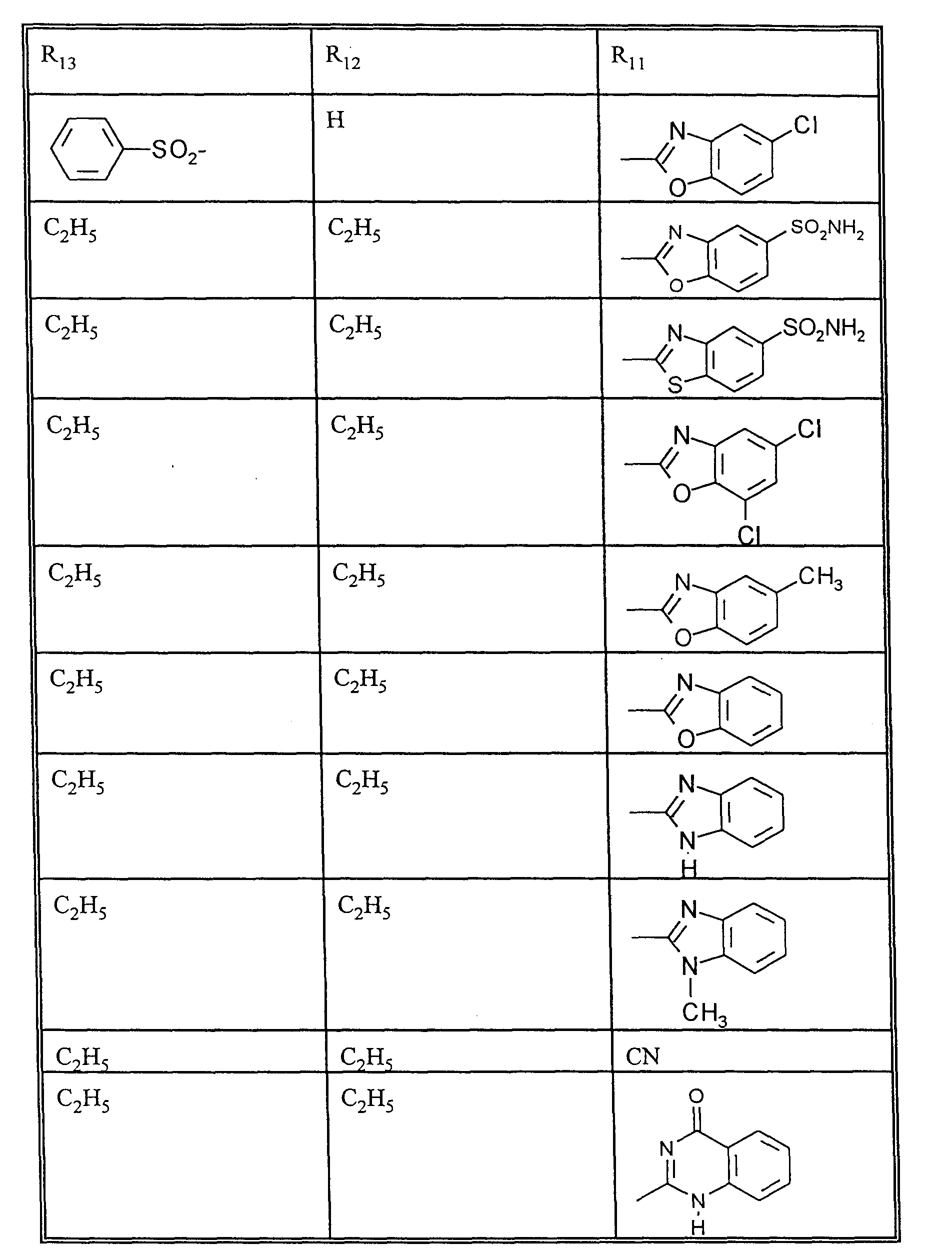

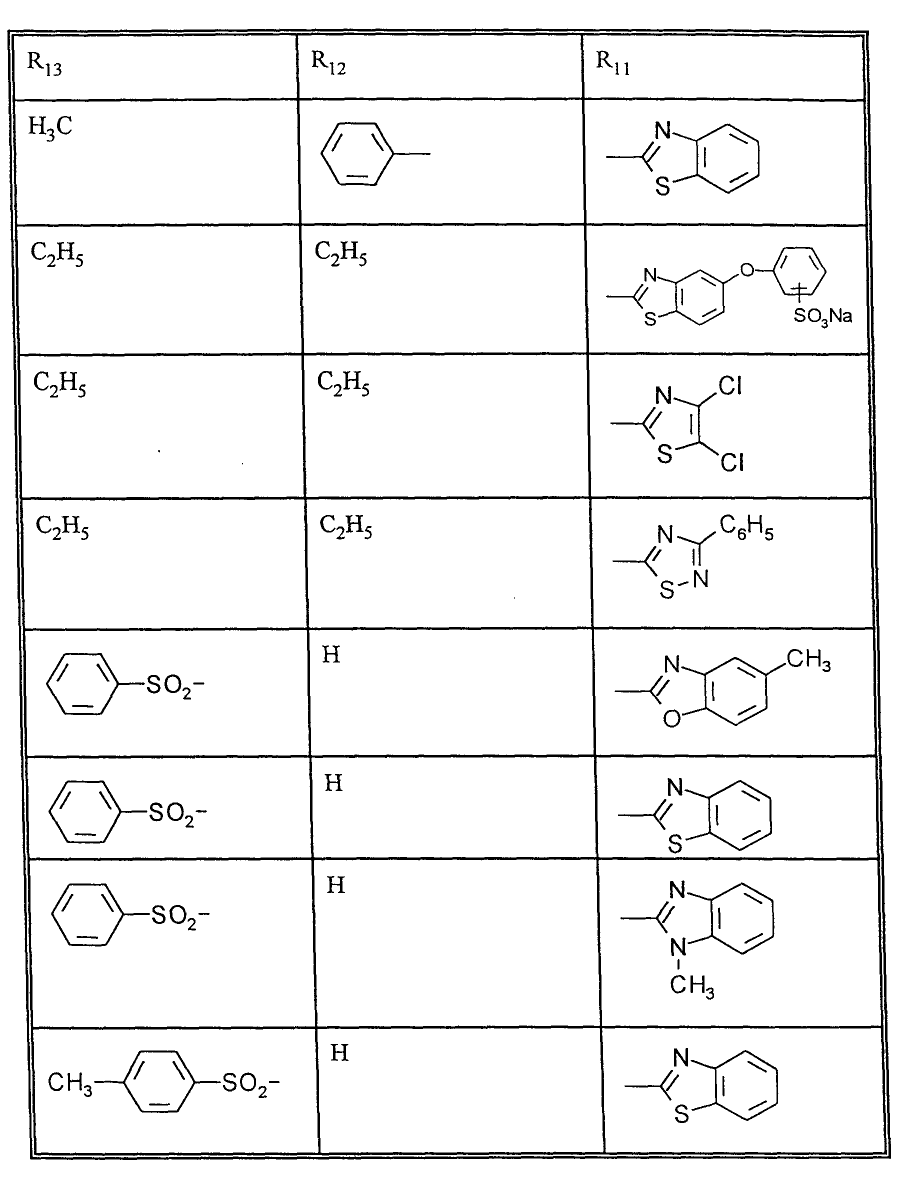

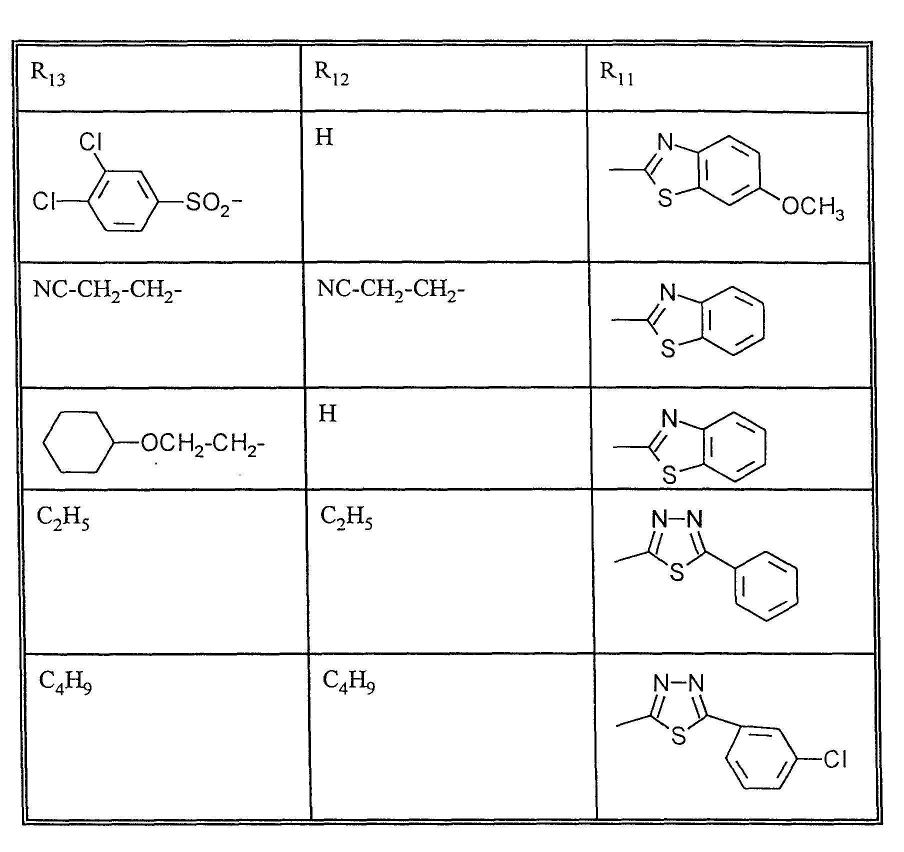

Analog zu Beispiel 1 und 2 lassen sich folgende Verbindungen herstellen:

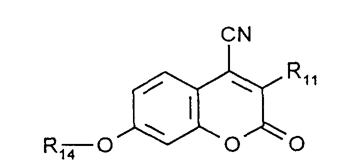

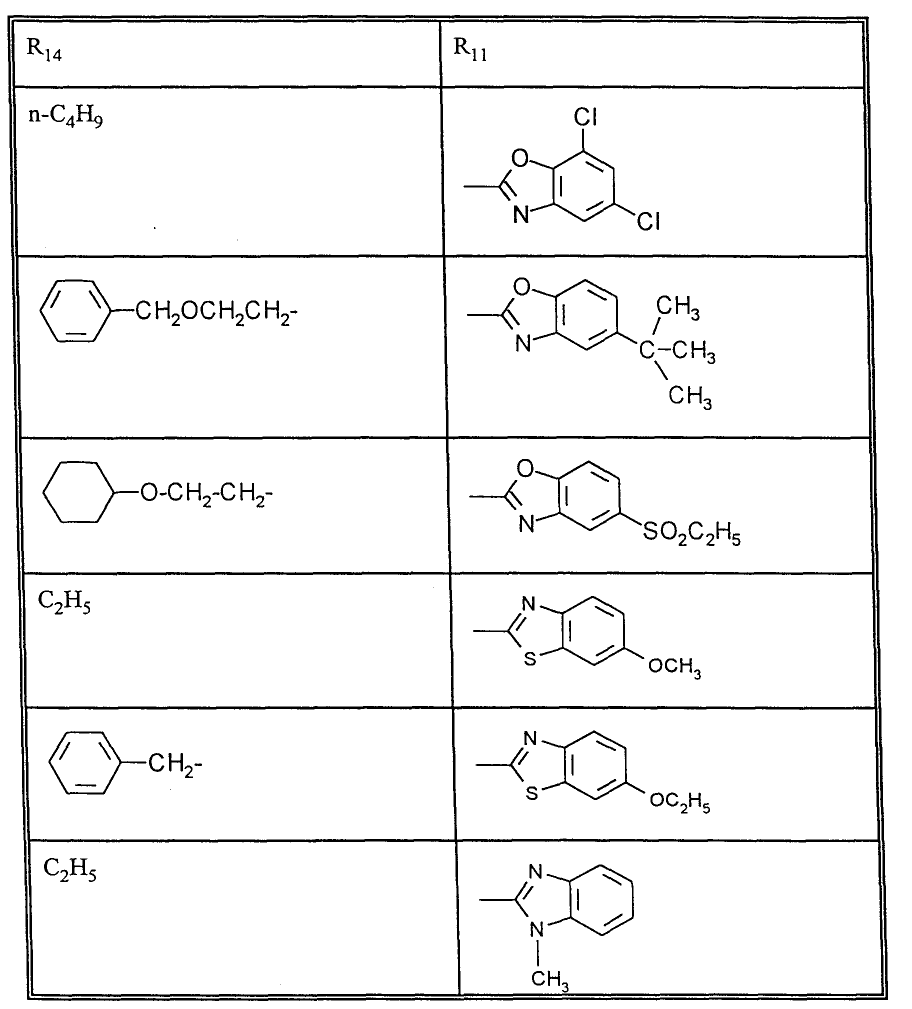

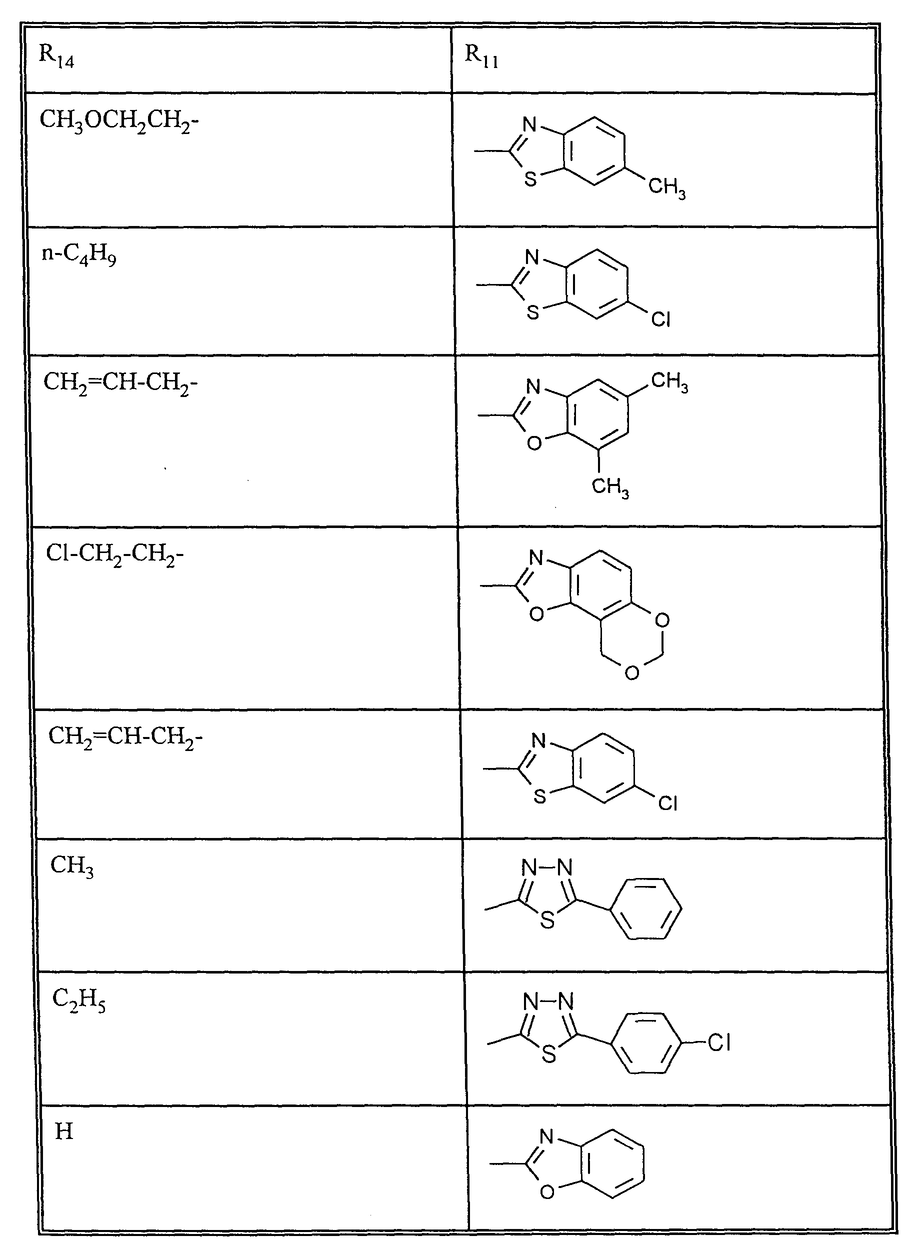

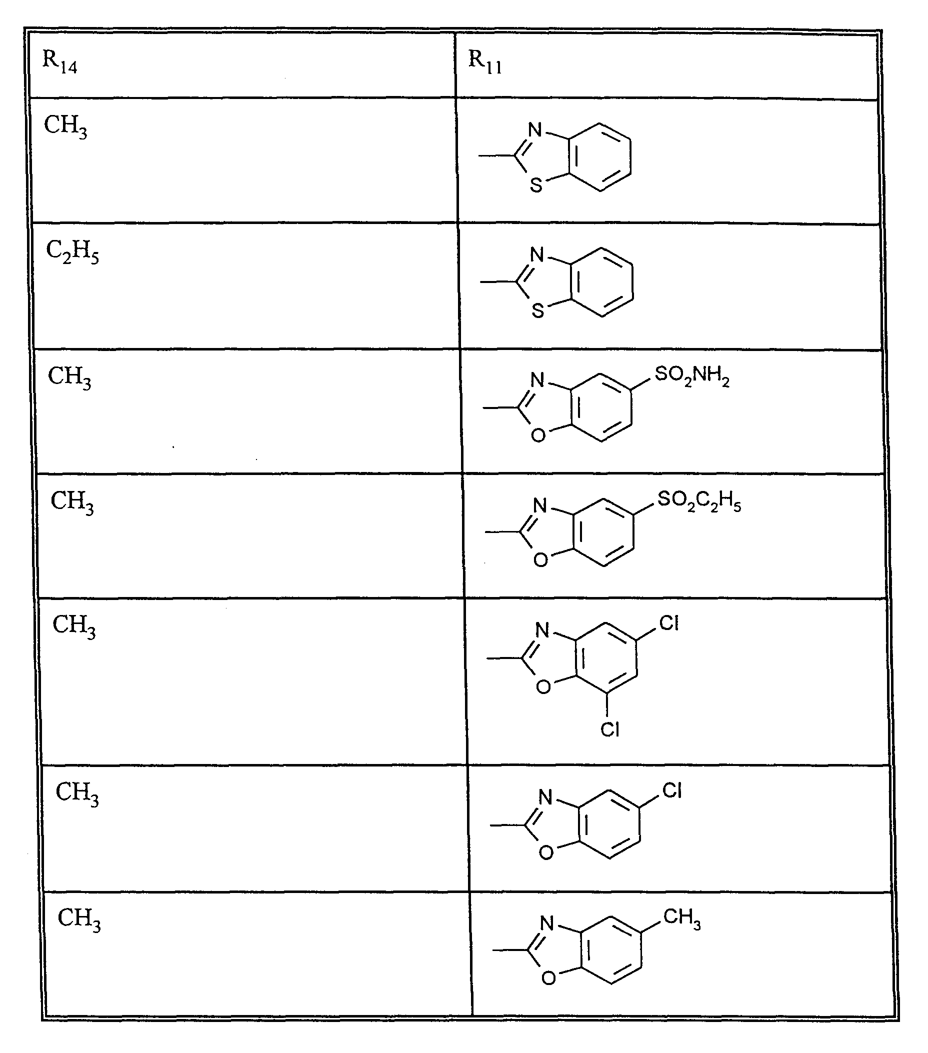

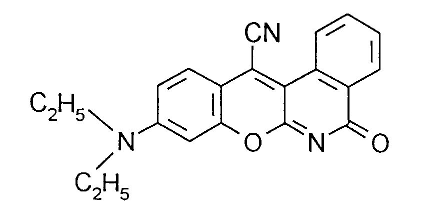

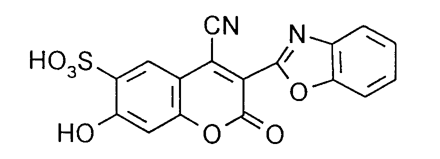

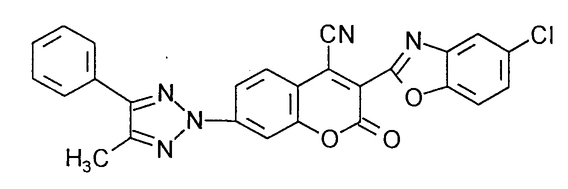

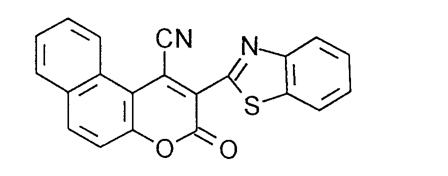

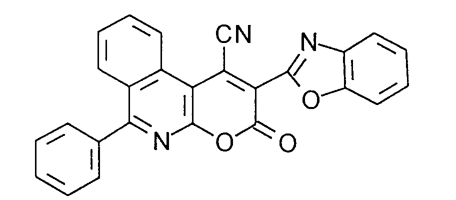

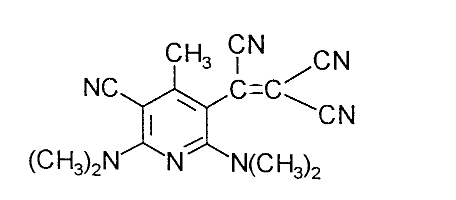

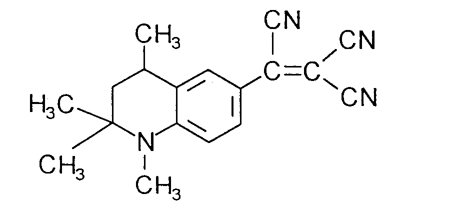

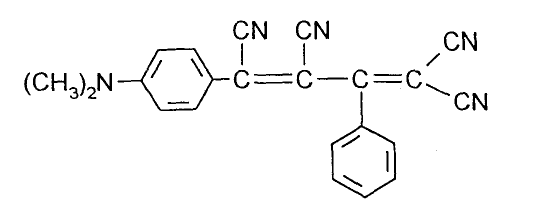

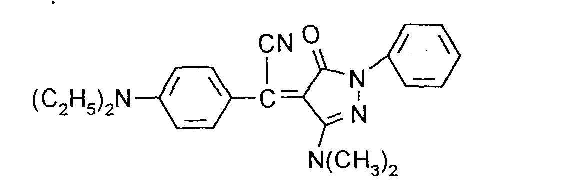

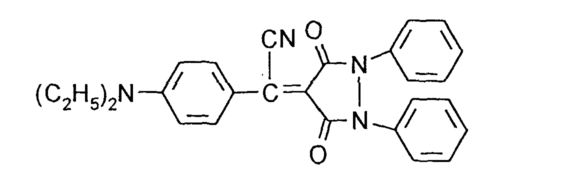

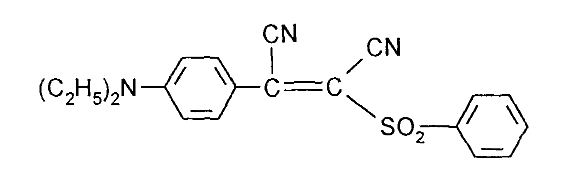

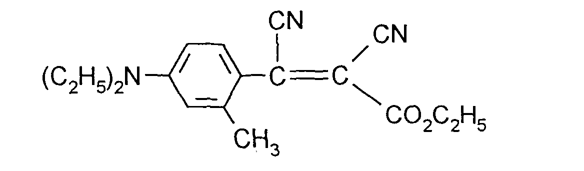

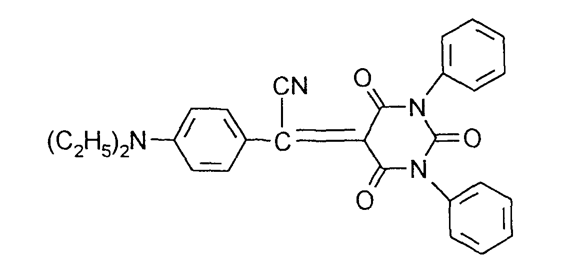

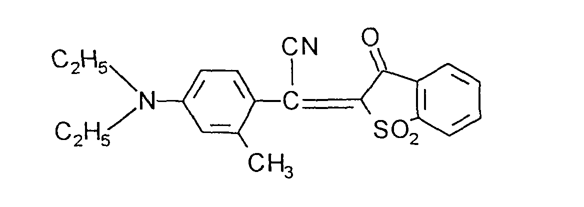

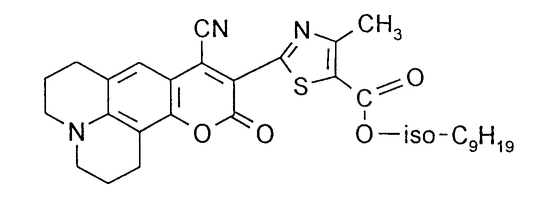

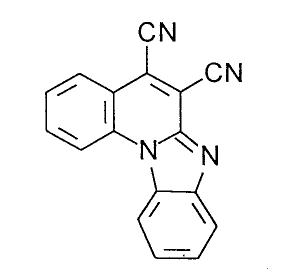

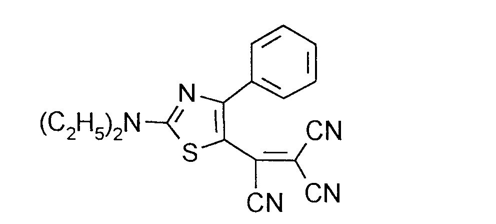

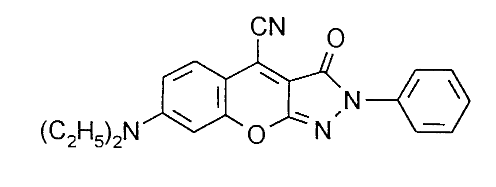

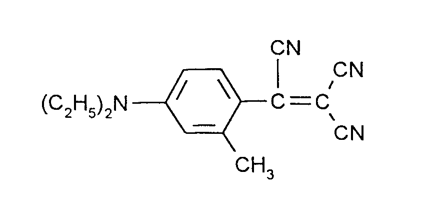

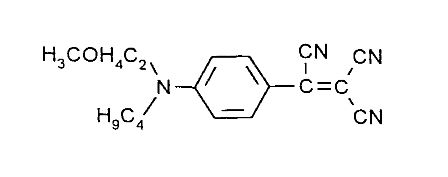

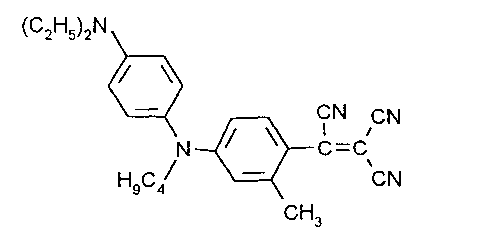

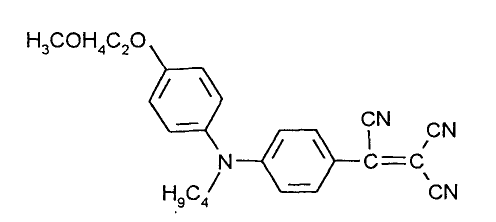

## Patentansprüche

1. Verfahren zur Herstellung von Verbindungen der Formel 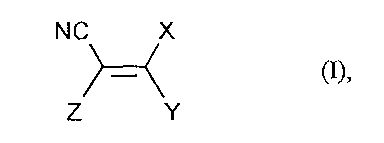 worin
X und Y unabhängig voneinander für einen elektronenanziehenden Rest stehen,
oder
X und Y gemeinsam mit dem Kohlenstoffatom, an welches sie gebunden sind, einen gegebenenfalls substituierten, heterocyclischen oder carbocyclischen Ring bilden,
Z für gegebenenfalls substituiertes gegebenenfalls anelliertes Aryl oder für einen gegebenenfalls substituierten gegebenenfalls anellierten Heterocyclus steht,
oder
Y und Z gemeinsam mit den Kohlenstoffatomen, an welche sie gebunden sind und unter Einschluß der Doppelbindung einen gegebenenfalls substituierten Heterocyclus bilden,
dadurch gekennzeichnet, daß man Verbindungen der Formel 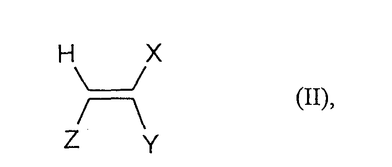 worin
X, Y und Z die oben angegebene Bedeutung haben,
mit einem Cyanid oder einer Cyanid-abgebenden Verbindung und anschließend mit einem von Br₂ verschiedenen Oxidationsmittel in Gegenwart von Halogeniden umsetzt.

2. Verfahren gemäß Anspruch 1, dadurch gekennzeichnet, daß man Verbindungen der Formel II mit einem Cyanid oder einer Cyanid-abgebenden Verbindung und anschließend mit einem von Br₂ verschiedenen Oxidationsmittel in Gegenwart von Halogeniden und Säure umsetzt.

3. Verfahren gemäß Anspruch 1, dadurch gekennzeichnet, daß X und Y unabhängig voneinander einen Rest bedeuten, dessen Hammett'sche Substituentenkonstante σ (para) > 0 ist.

4. Verfahren gemäß Anspruch 1, dadurch gekennzeichnet, daß Verbindungen der Formel (I) hergestellt werden,
worin
X für Cyan, -CO-OV₁ , 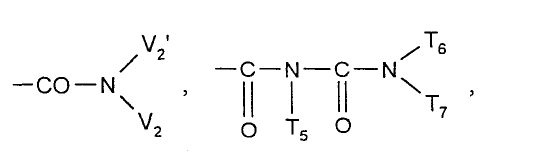 -CO-V₂, -SO₂V₁, -, 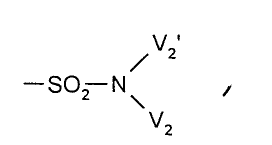 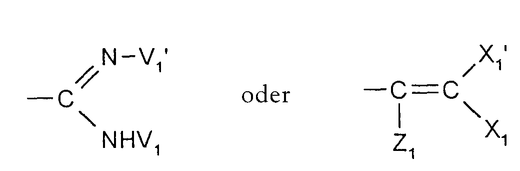 steht,
wobei
V₁ und V₁' unabhängig voneinander für Alkyl, Cycloalkyl, Alkenyl, Aralkyl oder Aryl stehen,
T₅, T₆ und T₇ unabhängig voneinander Wasserstoff, unsubstituiertes oder substituiertes Aryl, insbesondere Phenyl bedeuten,
V₂ und V₂' unabhängig voneinander für H oder V₁ stehen,
oder V₁ und V₁' bzw. V₂ und V₂' jeweils gemeinsam mit den Resten, an welche sie gebunden sind, für einen 5- oder 6-gliedrigen, gegebenenfalls substituierten ungesättigten, vorzugsweise aromatischen Heterocyclus stehen, der 1 bis 3 gleiche oder verschiedene Heteroatome aus der Reihe O, N, S enthält und der gegebenenfalls durch einen substituierten oder unsubstituierten Benzolring anelliert ist, oder für einen 5- oder 6-gliedrigen, gegebenenfalls substituierten ungesättigten Carbocyclus, vorzugsweise Phenyl, stehen,
X₁ und X₁' unabhängig voneinander für , 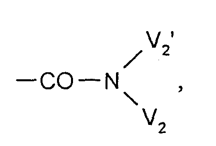 -CO-V₂ -SO₂V₁, 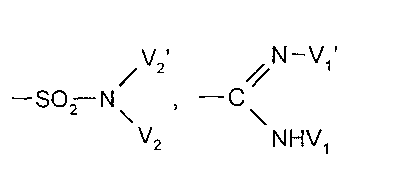 stehen, wobei
V₁, V₁', V₂ und V₂' die oben angegebene Bedeutung haben,
oder
X₁ und X₁' gemeinsam mit dem C-Atom, an das sie gebunden sind für einen 5- oder 6-gliedrigen, gegebenenfalls substituierten Heterocyclus, der 1 bis 2 N-Atome enthält und der gegebenenfalls
durch einen weiteren 5-gliedrigen ungesättigten Heterocyclus oder einen Benzolring anelliert ist, stehen,
Z₁ für gegebenenfalls substituiertes C₆-C₁₀-Aryl, das gegebenenfalls durch einen gesättigten oder ungesättigten Heterocyclus anelliert ist, oder für einen ungesättigten 5- oder 6-gliedrigen, gegebenenfalls substituierten, Heterocyclus, der 1 bis 4 gleiche oder verschiedene Heteroatome aus der Reihe O, N, S enthält und der durch einen Benzolring anelliert sein kann, steht,
oder
Z₁ und X₁ gemeinsam mit den Kohlenstoffatomen, an welche sie gebunden sind, und unter Einschluß der Doppelbindung einen gegebenenfalls substituierten und gegebenenfalls anellierten Heterocyclus oder einen aromatischen, carbocyclischen Ring, insbesondere Benzolring, bilden,
Y gleiches oder verschiedenes X bedeutet oder X und Y gemeinsam mit dem C-Atom, an das sie gebunden sind, für einen 5- oder 6-gliedrigen gegebenenfalls substituierten Heterocyclus, der 1 bis 2 N-Atome enthält, und der gegebenenfalls durch einen weiteren 5-gliedrigen ungesättigten Heterocyclus oder einen Benzolring anelliert ist, stehen,
Z für gegebenenfalls substituiertes C₆-C₁₀-Aryl, das gegebenenfalls durch einen gesättigten oder ungesättigten Heterocyclus anelliert ist, oder für einen ungesättigten 5- oder 6-gliedrigen gegebenenfalls substituierten Heterocyclus, der 1 bis 4 gleiche oder verschiedene Heteroatome aus der Reihe O, N, S enthält und der durch einen Benzoring anelliert sein kann, steht,
oder
Y und Z gemeinsam, unter Einschluß der Doppelbindung an welche sie gebunden sind, einen 6-gliedrigen, gegebenenfalls substituierten Heterocyclus bilden, welcher ein Heteroatom aus der Reihe O, N, S enthält und welcher gegebenenfalls durch einen substituierten oder unsubstituierten Benzolring anelliert ist,
wobei als Substituenten für den Carbocyclus und den Heterocyclus insbesondere Alkyl, Alkylsulfonyl, Aryl, Aralkyl, Alkenyl, -SO₂OR₁, Cycloalkyl, Alkoxy, Alkoxycarbonyl, Acyl, Halogen, Cyan, C=O, C=NH, jeweils gegebenenfalls substituiertes Carbonamid oder Sulfonamid, Nitro oder exocyclisches =O, =NH, in Frage kommen, und für den anellierten Benzolring als Substituenten in der Definition von X Alkyl, SO₂-Alkyl, gegebenenfalls -SO₂NR₁R₂, substituiertes Sulfonamid, Aryl, Aralkyl, Cycloalkyl, Alkoxy, Alkoxycarbonyl, Acyl, Halogen, Cyan, Nitro oder Alkenyl in Frage kommen,
und wobei als Substituenten für den aromatischen Carbocyclus oder den Heterocyclus in der Definition von Z 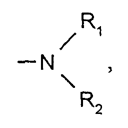 OR₁, NHCOR₁, NHSO₂R₁ in Frage kommen, wobei
R₁ und R₂ unabhängig voneinander für Wasserstoff, gegebenenfalls substituiertes Alkyl, Alkenyl, Cycloalkyl, Aralkyl oder Aryl stehen, oder R₁ und R₂ zusammen mit dem Stickstoffatom, an das sie gebunden sind, einen 5- bis 6-gliedrigen Heterocyclus mit 1 bis 3 Heteroatomen aus der Reihe O, N, S bilden.

5. Verfahren gemäß Anspruch 1, dadurch gekennzeichnet, daß es sich bei den Verbindungen (I) um solche der Formel 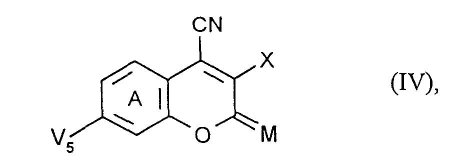 handelt, in welcher
V₅ für 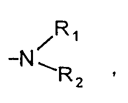 OR₁, NHCOR₁ oder NHSO₂R₁ steht,
R₁ und R₂ unabhängig voneinander für Wasserstoff, gegebenenfalls substituiertes Alkyl, Cycloalkyl, Aralkyl oder Aryl stehen, oder
R₁ und R₂ zusammen mit dem Stickstoffatom, an das sie gebunden sind, und gegebenenfalls unter Einschluß weiterer Heteroatome einen 5- bis 7-gliedrigen Heterocyclus bilden,
oder einer der Reste R₁ oder R₂ mit einem zur Aminogruppe o-ständigen Kohlenstoffatom des Ringes A einen ankondensierten, gesättigten, gegebenenfalls substituierten 5- oder 6-Ring bildet,
M für =NR₃, =NCOR₄, 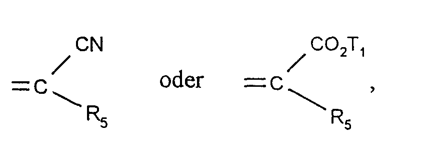 insbesondere für =NH oder =O steht, wobei
R₃ für Alkyl oder gegebenenfalls substituiertes Phenyl,
R₄ für gegebenenfalls substituiertes Alkyl, Aralkyl, Aryl, Vinyl, Alkoxy, Phenoxy oder Amino und
R₅ für Carbalkoxy, Cyano oder gegebenenfalls substituiertes Carbonamid steht, oder
R₅ zusammen mit X die restlichen Glieder eines ungesättigten 6-gliedrigen, gegebenenfalls anellierten N-Heterocyclus bildet,
und
X die in Anspruch 1 angegebene Bedeutung hat.

6. Verfahren gemäß Ansprüchen 1 bis 5, dadurch gekennzeichnet, daß als Cyanid eine organische oder anorganische Cyanverbindung oder eine Cyanid-abspaltende Verbindung oder ein Gemisch dieser Verbindungen eingesetzt wird.

7. Verfahren gemäß Ansprüchen 1 bis 6, dadurch gekennzeichnet, daß als Lösungsmittel mindestens ein polares Lösungsmittel, gegebenenfalls in Kombination mit einem Phasentransferkatalysator, eingesetzt wird.

8. Verfahren gemäß Ansprüchen 1 bis 7, dadurch gekennzeichnet, daß als Oxidationsmittel solche mit einem chemischen Potential von größer 0,1 eV, vorzugsweise Wasserstoffperoxid, eingesetzt wird.

9. Verfahren gemäß Ansprüchen 1 bis 8, dadurch gekennzeichnet, daß als Halogenide organische und/oder anorganische Verbindungen des Chlors, Broms oder Iods in Mengen von 0,1 bis 200 mol-%, bezogen auf das Edukt (II) eingesetzt werden.

10. Verfahren gemäß Ansprüchen 2 bis 9, dadurch gekennzeichnet, daß man als Säure eine Carbon- oder Mineralsäure einsetzt.

11. Verfahren gemäß Ansprüchen 2 bis 10, dadurch gekennzeichnet, daß man die Lösung des Umsetzungsproduktes von (II) mit dem Cyanid zunächst mit der Säure versetzt und dann nacheinander das Halogenid und das Oxidationsmittel zufügt.

12. Verfahren gemäß Ansprüchen 2 bis 10, dadurch gekennzeichnet, daß man Säure, Halogenid und Oxidationsmittel in dem Lösungsmittel vorlegt und dann das Umsetzungsprodukt von (II) mit dem Cyanid zufügt.

## Claims

1. Process for the preparation of compounds of the formula 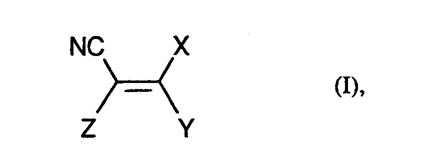 wherein
X and Y independently of one another represent an electron-attracting radical,
or
X and Y, together with the carbon atom to which they are bonded, form an optionally substituted, heterocyclic or carbocyclic ring,
Z represents optionally substituted, optionally fused aryl or represents an optionally substituted, optionally fused heterocyclic radical,
or
Y and Z, together with the carbon atoms to which they are bonded and including the double bond, form an optionally substituted heterocyclic radical,
characterized in that compounds of the formula 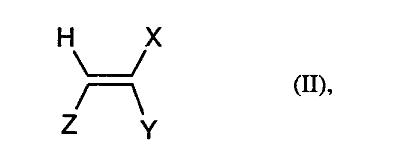 wherein
X, Y and Z have the abovementioned meaning,
are reacted with a cyanide or a cyanide-donating compound and then with an oxidizing agent other than Br₂ in the presence of halides.

2. Process according to Claim 1, characterized in that compounds of the formula II are reacted with a cyanide or a cyanide-donating compound and then with an oxidizing agent other than Br₂ in the presence of halides and acid.

3. Process according to Claim 1, characterized in that X and Y independently of one another denote a radical of which the Hammett substituent constant σ (para) > 0.

4. Process according to Claim 1, characterized in that compounds of the formula (I)
wherein
X represents cyano, -CO-OV₁, 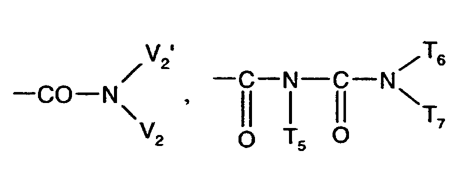 -CO-V₂, -SO₂V₁, -, 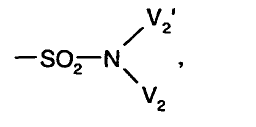 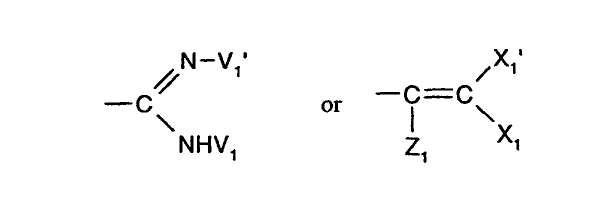 wherein
V₁ and V₁' independently of one another represent alkyl, cycloalkyl, alkenyl, aralkyl or aryl,
T₅, T₆ and T₇ independently of one another denote hydrogen or unsubstituted or substituted aryl, in particular phenyl,
V₂ and V₂' independently of one another represent H or V₁,
or V₁ and V₁' or V₂ and V₂', in each case together with the radicals to which they are bonded, represent a 5- or 6-membered, optionally substituted unsaturated, preferably aromatic heterocyclic radical which contains 1 to 3 identical or different hetero atoms from the series consisting of O, N and S and is optionally fused by a substituted or unsubstituted benzene ring, or represent a 5- or 6-membered, optionally substituted unsaturated carbocyclic radical, preferably phenyl,
X₁ and X₁' independently of one another represent 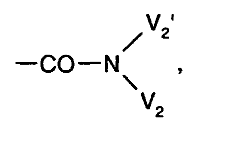 -CO-V₂, -SO₂V₁, 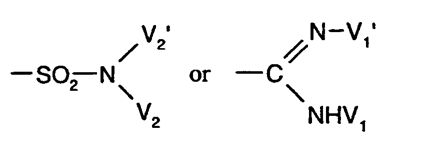 wherein
V₁, V₁', V₂ and V₂' have the abovementioned meaning,
or
X₁ and X₁', together with the C atom to which they are bonded, represent a 5- or 6-membered, optionally substituted heterocyclic radical which contains 1 to 2 N atoms and is optionally fused by a further 5-membered unsaturated heterocyclic radical or a benzene ring,
Z₁ represents optionally substituted C₆-C₁₀-aryl, which is optionally fused by a saturated or unsaturated heterocyclic radical, or represents an unsaturated 5- or 6-membered, optionally substituted, heterocyclic radical which contains 1 to 4 identical or different hetero atoms from the series consisting of O, N and S and can be fused by a benzene ring,
or
Z₁ and X₁, together with the carbon atoms to which they are bonded and including the double bond, form an optionally substituted and optionally fused heterocyclic radical or an aromatic carbocyclic ring, in particular a benzene ring,
Y is identical to or different from X, or X and Y, together with the C atom to which they are bonded, represent a 5- or 6-membered optionally substituted heterocyclic radical which contains 1 to 2 N atoms and which can optionally be fused by a further 5-membered unsaturated heterocyclic ring or a benzene ring,
Z represents optionally substituted C₆-C₁₀-aryl, which is optionally fused by a saturated or unsaturated heterocyclic radical, or represents an unsaturated 5- or 6-membered optionally substituted heterocyclic radical which contains 1 to 4 identical or different hetero atoms from the series consisting of O, N and S and can be fused by a benzo ring,
or
Y and Z together, including the double bond to which they are bonded, form a 6-membered, optionally substituted heterocyclic radical which contains a hetero atom from the series consisting of O, N and S and is optionally fused by a substituted or unsubstituted benzene ring,
where possible substituents for the carbocyclic and the heterocyclic radical are, in particular, alkyl, alkylsulphonyl, aryl, aralkyl, alkenyl, -SO₂OR₁, cycloalkyl, alkoxy, alkoxycarbonyl, acyl, halogen, cyano, C=O, C=NH, in each case optionally substituted carboxamide or sulphonamide, nitro or exocyclic =O or =NH, and
possible substituents within the definition of X for the fused benzene ring are alkyl, SO₂-alkyl, optionally -SO₂NR₁R₂, substituted sulphonamide aryl, aralkyl, cycloalkyl, alkoxy, alkoxycarbonyl, acyl, halogen, cyano, nitro or alkenyl,
and where possible substituents for the aromatic carbocyclic radical or the heterocyclic radical in the definition of Z are 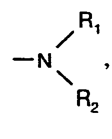 OR₁, NHCOR₁ and NHSO₂R₁
in which
R₁ and R₂ independently of one another represent hydrogen, optionally substituted alkyl, alkenyl, cycloalkyl, aralkyl or aryl, or R₁ and R₂, together with the nitrogen atom to which they are bonded, form a 5- to 6-membered heterocyclic radical having 1 to 3 hetero atoms from the series consisting of O, N and S,
are prepared.

5. Process according to Claim 1, characterized in that the compounds (I) are those of the formula 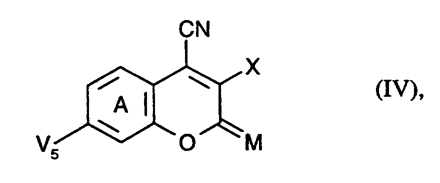 in which
V₅ represents 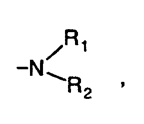 OR₁, NHCOR₁ or NHSO₂R₁,
R₁ and R₂ independently of one another represent hydrogen, optionally substituted alkyl, cycloalkyl, aralkyl or aryl, or
R₁ and R₂, together with the nitrogen atom to which they are bonded and optionally including further hetero atoms, form a 5- to 7-membered heterocyclic radical,
or one of the radicals R₁ or R₂, with a carbon atom of ring A in the o-position relative to the amino group, forms a fused-on, saturated, optionally substituted 5- or 6-membered ring,
M represents =NR₃, =NCOR₄, 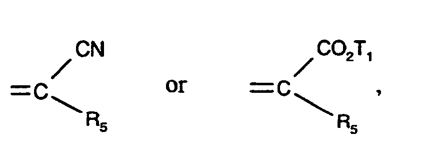 in particular =NH or =O, wherein
R₃ represents alkyl or optionally substituted phenyl,
R₄ represents optionally substituted alkyl, aralkyl, aryl, vinyl, alkoxy, phenoxy or amino and
R₅ represents carbalkoxy, cyano or optionally substituted carboxamide, or
R₅, together with X, forms the remaining members of an unsaturated 6-membered, optionally fused N-heterocyclic radical,
and
X has the meaning given in Claim 1.

6. Process according to Claims 1 to 5, characterized in that an organic or inorganic cyano compound or a compound which splits off cyanide or a mixture of these compounds is employed as the cyanide.

7. Process according to Claims 1 to 6, characterized in that at least one polar solvent, if appropriate in combination with a phase transfer catalyst, is employed as the solvent.

8. Process according to Claims 1 to 7, characterized in that the oxidizing agent employed is one having a chemical potential of greater than 0.1 eV, preferably hydrogen peroxide.

9. Process according to Claims 1 to 8, characterized in that organic and/or inorganic compounds of chlorine, bromine or iodine are employed as halides in amounts of 0.1 to 200 mol %, based on the educt (II).

10. Process according to Claims 2 to 9, characterized in that a carboxylic or mineral acid is employed as the acid.

11. Process according to Claims 2 to 10, characterized in that the acid is first added to the solution of the reaction product of (II) with the cyanide and the halide and the oxidizing agent are then added in succession.

12. Process according to Claims 2 to 10, characterized in that the acid, halide and oxidizing agent are initially introduced into the reaction vessel in the solvent and the reaction product of (II) with the cyanide is then added.

## Revendications

1. Procédé de préparation de composés de formule 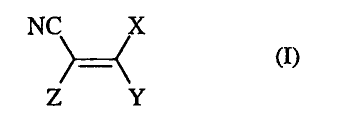 dans laquelle
X et Y représentent chacun, indépendamment l'un de l'autre, un substituant électrophile,
ou bien
X et Y forment ensemble et avec l'atome de carbone auquel ils sont reliés, un noyau hétérocyclique ou carbocyclique éventuellement substitué,
Z représente un groupe aryle éventuellement substitué et éventuellement condensé ou un hétérocycle éventuellement substitué et éventuellement condensé,
ou bien
Y et Z forment ensemble et avec les atomes de carbone auxquels ils sont reliés, avec inclusion de la double liaison, un hétérocycle éventuellement substitué,
caractérisé en ce que l'on fait réagir des composés de formule 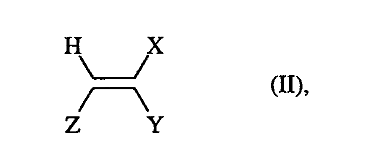 dans laquelle
X, Y et Z ont les significations indiquées ci-dessus,
avec un cyanure ou un composé fournissant un cyanure puis avec un agent oxydant autre que Br₂, en présence d'halogénures.

2. Procédé selon la revendication 1, caractérisé en ce que l'on fait réagir des composés de formule (II) avec un cyanure ou un composé fournissant un cyanure puis avec un agent oxydant autre que Br₂ en présence d'un halogénure et d'un acide.

3. Procédé selon la revendication 1, caractérisé en ce que X et Y représentent chacun, indépendamment l'un de l'autre, un substituant dont la constante de substituant de Hammett σ (para) est supérieure à 0.

4. Procédé selon la revendication 1, caractérisé en ce que l'on prépare des composés de formule (I) dans laquelle
X représente un groupe cyano, -CO-OV₁, 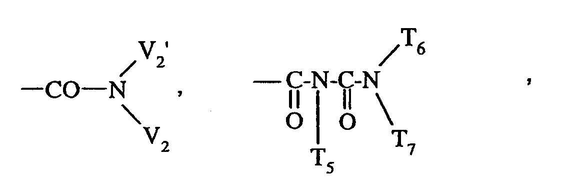 -CO-V₂, -SO₂V₁, -, 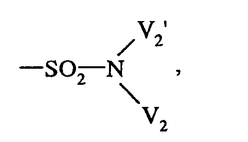 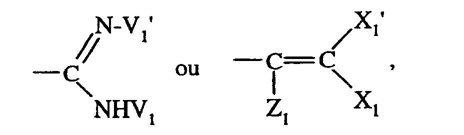
dans lequel
V₁ et V₁' représentent chacun, indépendamment l'un de l'autre, un groupe alkyle, cycloalkyle, alcényle, aralkyle ou aryle,
T₅, T₆ et T₇ représentent chacun, indépendamment les uns des autres, l'hydrogène, un groupe aryle substitué ou non, plus spécialement phényle,
V₂ et V₂' indépendamment l'un de l'autre, représentent l'hydrogène ou ont l'une des significations indiquées pour V₁,
ou bien V₁ et V₁' d'une part, V₂ et V₂' d'autre part, forment ensemble et avec les groupes auxquels ils sont reliés, un hétérocycle insaturé, de préférence aromatique, à 5 ou 6 chaînons, éventuellement substitué, qui contient 1 à 3 hétéroatomes identiques ou différents choisis parmi O, N et S et qui le cas échéant est condensé avec un noyau benzénique substitué ou non, ou bien un carbocycle insaturé à 5 ou 6 chaînons, éventuellement substitué, de préférence un cycle phényle,
X₁ et X₁' représentent chacun, indépendamment l'un de l'autre 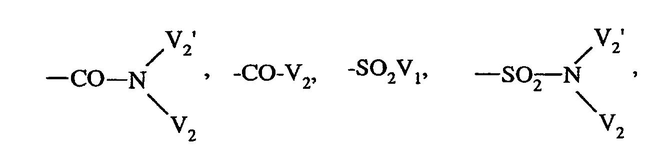 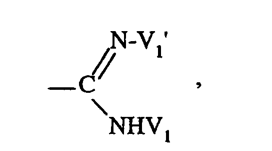 dans lesquels
V₁, V₁', V₂ et V₂' ont les significations indiquées ci-dessus,
ou bien
X₁ et X₁' forment ensemble et avec l'atome de carbone auquel ils sont reliés un hétérocycle à 5 ou 6 chaînons éventuellement substitué, qui contient 1 à 2 atomes d'azote et qui le cas échéant est condensé avec un autre hétérocycle insaturé à 5 chaînons ou avec un cycle benzénique,
Z₁ représente un groupe aryle en C₆-C₁₀ éventuellement substitué, éventuellement condensé avec un hétérocycle saturé ou insaturé, ou bien un hétérocycle insaturé à 5 ou 6 chaînons, éventuellement substitué, qui contient 1 à 4 hétéroatomes identiques ou différents choisis parmi O, N, S et qui peut être condensé avec un noyau benzénique,
ou bien
Z₁ et X₁ forment ensemble et avec les atomes de carbone auxquels ils sont reliés et avec inclusion de la double liaison un hétérocycle éventuellement substitué et éventuellement condensé ou un cycle aromatique homogène, en particulier un cycle benzénique,
Y a les mêmes significations que X mais il est identique à X ou différent de X, ou bien X et Y forment ensemble et avec l'atome de carbone auquel ils sont reliés, un hétérocycle à 5 ou 6 chaînons, éventuellement substitué, qui contient 1 à 2 atomes d'azote et qui le cas échéant est condensé avec un autre hétérocycle insaturé à 5 chaînons ou avec un cycle benzénique,
Z représente un groupe aryle en C₆-C₁₀ éventuellement substitué, éventuellement condensé avec un hétérocycle saturé ou insaturé, ou bien un hétérocycle insaturé à 5 ou 6 chaînons, éventuellement substitué, qui contient 1 à 4 hétéroatomes identiques ou différents choisis parmi O, N, S, et qui peut être condensé avec un cycle benzénique,
ou bien
Y et Z forment ensemble, avec inclusion de la double liaison à laquelle ils sont reliés, un hétérocycle à 6 chaînons, éventuellement substitué, qui contient un hétéroatome choisi parmi O, N, S et qui le cas échéant est condensé avec un cycle benzénique substitué ou non,
les substituants du carbocycle et de l'hétérocycle étant en particulier des groupes alkyle, alkylsulfonyle, aryle, aralkyle, alcényle, -SO₂OR₁, cycloalkyle, alcoxy, alcoxycarbonyle, acyle, des halogènes, des groupes cyano, C=O, C=NH, carboxamide ou sulfonamide chacun d'eux éventuellement substitué, nitro ou =O, =NH exocyclique, et les substituants d'un cycle benzénique condensé, répondant à la définition de X, sont des groupes alkyle, SO₂-alkyle, sulfonamide éventuellement substitué -SO₂NR₁R₂, aryle, aralkyle, cycloalkyle, alcoxy, alcoxycarbonyle, acyle, des halogènes, des groupes cyano, nitro ou alcényle,
les substituants d'un carbocycle aromatique ou de l'hétérocycle répondant à la définition de Z, consistant en 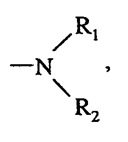 OR₁, NHCOR₁, NHSO₂R₁ dans lesquels
R₁ et R₂ représentent chacun, indépendamment l'un de l'autre, l'hydrogène, un groupe alkyle, alcényle, cycloalkyle, aralkyle ou aryle éventuellement substitué, ou bien R₁ et R₂ forment ensemble et avec l'atome d'azote auquel ils sont reliés, un hétérocycle à 5 ou 6 chaînons contenant 1 à 3 hétéroatomes choisis parmi O, N, S.

5. Procédé selon la revendication 1, caractérisé en ce que les composés (I) répondent à la formule 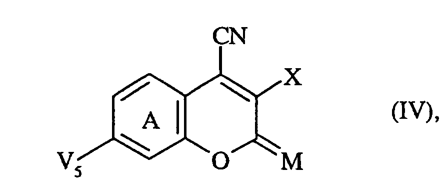 dans laquelle
V₅ représente 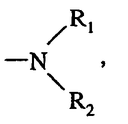 OR₁, NHCOR₁ ou NHSO₂R₁,
R₁ et R₂ représentent chacun, indépendamment l'un de l'autre, l'hydrogène, un groupe alkyle, cycloalkyle, aralkyle ou aryle éventuellement substitué, ou bien R₁ et R₂ forment ensemble et avec l'atome d'azote auquel ils sont reliés et le cas échéant avec inclusion d'autres hétéroatomes, un hétérocycle de 5 à 7 chaînons, ou bien
l'un des groupes R₁ ou R₂ forme, avec un atome de carbone du cycle A en position ortho du groupe amino, un cycle condensé, saturé, éventuellement substitué, à 5 ou 6 chaînons,
M représente NR₃, =NCOR₄, 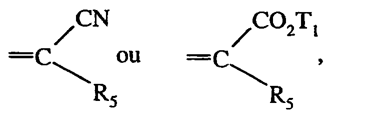 plus spécialement =NH ou =O,
R₃ représentant un groupe alkyle ou un groupe phényle éventuellement substitué,
R₄ représentant un groupe alkyle, aralkyle, aryle, vinyle, alcoxy, phénoxy ou amino éventuellement substitué et
R₅ représente un groupe carbalcoxy, cyano ou un groupe carboxamide éventuellement substitué, ou bien
R₅ forme avec X les chaînons restants d'un hétérocycle azoté insaturé à 6 chaînons, éventuellement condensé,
et
X a les significations indiquées dans la revendication 1.

6. Procédé selon les revendications 1 à 5, caractérisé en ce que le cyanure utilisé est un dérivé cyané organique ou inorganique ou un composé libérant un cyanure ou un mélange de tels composés.

7. Procédé selon les revendications 1 à 6, caractérisé en ce que l'on utilise en tant que solvant au moins un solvant polaire, le cas échéant en combinaison avec un catalyseur à transfert de phase.

8. Procédé selon les revendications 1 à 7, caractérisé en ce que l'on utilise un agent oxydant ayant un potentiel chimique supérieur à 0,1 eV, de préférence le peroxyde d'hydrogène.

9. Procédé selon les revendications 1 à 8, caractérisé en ce que les halogénures utilisés sont des composés organique et/ou inorganiques du chlore, du brome ou de l'iode, qu'on utilise en quantité de 0,1 à 200 mol % par rapport au composant de départ (II).

10. Procédé selon les revendications 2 à 9, caractérisé en ce que l'acide utilisé est un acide carboxylique ou un acide minéral.

11. Procédé selon les revendications 2 à 10, caractérisé en ce que, à la solution du produit de réaction (II) et du cyanure, on ajoute d'abord l'acide puis, successivement l'halogénure et l'agent oxydant.

12. Procédé selon les revendications 2 à 10, caractérisé en ce que l'on introduit d'abord l'acide, l'halogénure et l'agent oxydant dans le solvant puis on ajoute le produit de réaction de (II) et du cyanure.
